# EUROPEAN PATENT APPLICATION

(11) **EP 2 127 535 A1**
(43) Date of publication of application: **02.12.2009**
(21) Application number: 08460017.0
(22) Date of filing: 21.05.2008
(51) Int. Cl.: A23G 3/48, A61K 8/02, A61K 8/97, A61Q 11/00, A23G 4/06, A23G 3/38

(54) **Flaxseed product for cleaning the tongue, the method of the production of the product for cleaning the tongue, use of flaxseed for the product**

(71) Applicant: Instytut Wlokien Naturalnych (Institut of Natural Fibres), 60-630 - (PL)
(72) Inventor: Kozlowska, Jadwiga, 61-606 Poznan (PL); Kregielczak, Agnieszka, 61-680 Poznan (PL); Talarczyk, Alina, 63-700 Krotoszyn (PL); Makota, Malgorzala, 63-700 Krotoszyn (PL)
(74) Representative: Twardowska, Aleksandra

(57) **Abstract**

The subjects of the invention are the product for cleaning the tongue, the method of its production and using flaxseed. More specifically the solution involves food or pharmaceutical products such as candies or lozenges for sucking with addition of flaxseed, the method of their production and using flaxseed for producing a refreshing product for cleaning the tongue of bacterial fur, proving at the same time mechanical cleaning of the tongue and hindering formation of bacterial fur and being a dietary supplement or pharmaceutical product by physiological and nutritional effects.

## Description

The subject matters of the invention is a product with flaxseed for cleaning the tongue, the method of production of the product and use of flaxseed for producing refreshing product for cleaning the tongue from bacterial residue,, by simultaneously mechanical cleaning of the tongue and inhibiting formation of bacterial fur on the tongue.

Bad breath, referred in the literature as oral malodor, fetor ex ore or halitosis is a quite common symptom of complex etiology. For 90% of the population suffering from bad breath the causes are located within oral cavity. One of the main causes of bad breath and discomfort that it brings about is organic fur forming on the tongue. Some share of the fur are bacteria that produce volatile sulphur components characterized with unpleasant odor (VSC - *volatile sulfur components)* [Kregielczak A. Nieprzyjemny oddech z jamy ustnej- etiologia, patogeneza, leczenie. Poznańska Stomatologia; 2001:135139]. The direct correlation between bad breath and the presence of the fur was shown by Boever et al. [Boever E, Loesche WJ. Assesing the contribution of anaerobic microflora of the tongue to oral malador. JADA 1995; 126: 1384-1394]*.* In microbiological studies on the fur on the tongue of the patients suffering from bad breath Gram negative bacteria have been isolated, which are able to produce it their metabolic processes hydrogen sulphide and methyl mercaptane(*Treponema denticola, Porphyromonas gingivialis, Pretovella intermedia* and *Bacteroides forsythus).* There is a direct link between the bad breath and produced by bacteria hydrogen sulphide and methyl mercaptane [Replogle WH, Bebe DK; Halitosis; American Family Physician; 1996: 53 (4): 1215-1218]*.* Gram negative bacteria populating the ridge of the tongue metabolize the remains of food, while the metabolic products of decomposition of both exogenic and endogenic proteins (from exfoliated epithelium cells, leucocytes, saliva and blood) are volatile components of unpleasant odor.

It has been proved that both hydrogen sulphide and methyl mercaptane can penetrate the epithelium cells, disturbing their metabolism and therefore promote parodontium diseases. These are the compounds that influence collagen synthesis and degradation and stimulate IL-1 interleukin production. It is a cytokine that induces E2 prostaglandin production, which in turn facilitates bone resorption [Walter S SM. On the transformation of sulfur- containing amino acids and peptides to volatile sulfur compounds(VSC) in human mouth; Eur J Oraj Sci.; 1997; (105): 534-537].

It was proven that removing the fur from the tongue lowers the amount of sulphur volatile components in exhaled air [Yaegaki K, Sanada K. Volatile sulfur compounds in mouth air from clinically healthy parcipitans and patients with periodontal disease; J. Periodon; Res 1992; 27:233-238].

The available literature on the subject differentiates between non-pathological and pathological causes of bad breath [Replogle WH, Bebe DK; Halitosis; American Family Physician; 1996:53(4):1215-1218]*.* Non-pathological causes include reduced saliva production during sleep, what results in increase of Gram negative bacteria producing gases of unpleasant odor. In elderly patients decrease in saliva production results from degenerative changes within saliva glands. Breath of such patients is widely believed as less pleasant than in younger patients. Also higher pH of saliva promotes higher number of bacteria and transformation of proteins into volatile sulphur compounds *[*Kregielczak A., Nieprzyjemny oddech z jamy ustnej - etiologia, patogeneza, leczenie; Poznańska Stomatologia; 2001:135139].

The cause of unpleasant odor can also stem from hunger and tobacco smoking. It is known that some kinds of food such as onions, garlic or alcohol bring about unpleasant odor in oral cavity, as these components stay in saliva for up to 72 hours [Scully C., El-Maaytah M, Porter SR, Greenman J. Breath odor: etiopathogenesis, assessment and management. Eur J Oral Sci. 1997; 105: 287-293].

It was confirmed that the level of the volatile compounds drops significantly two hours after the meal and cleaning the ridge surface of the tongue is one of the factors that help in removing the components. It was proven that mouth rinses containing chemical agents such as chlorhexidine, bicarbonates, zincum chloride or cetylpirydine can lower the level of bad breath. Zinc and triclosan in the rinses and toothpastes show the same effect [Pitts G, Brogdon C, Hu L, Masurat T, Pianotti R, Schumann P.; Mechanism of an antiseptic, anti odor mouthwash.; J Dent Res.; 1983; 62:738-742]*.* The intensity of bad breath can be lowered by chewing gums containing katechines. It is also recommended to use so called oxidizing lozenges for sucking and rinses, when sucking the lozenges reduces the odor that comes from the ridge part of the tongue.

The patent application P-350925 (publ. 12.10.2000) the methods and compositions were described that improve the durability of flavor in chewing gums. The solution is provided by matrix composition containing hydroxypropylocellulose hardened with multifunctional carboxylane in order to create a matrix of lower solubility in water than the original cellulosic material. The matrix can be ground, can have flavor introduced inside and can be used in chewing gum compositions, in which it facilitates prolonged release of flavor.

In the patent application P-377915 (publ. 14.05.2007) a food product based on flaxseed was described that contains flaxseed, preferably fragmented or crushed and preferably partially or completely defatted and seeds and/or husks of psyllium (*Plantago ovata* and/or *Plantago isphagula*)*,* preferably fragmented, whereas the content of seed and/or husks of psyllium (*Plantago ovata* and/or *Plantago isphagula*) is 1% to 50% of the weight, preferably from 5% to 20% of weight of flaxseed content. The mixture contains preservatives and/or sweeteners, colorants, food flavorings or other substances improving taste and smell and an addition of probiotic bacteria and/or prebiotic components, preferably inulin.

In the patent application P-363792 (publ. 30.05.2005) ground flaxseed of fruit flavor, containing defatted ground flaxseed, containing additionally sweetening component and fruit flavor, while the defatted ground flaxseed comprise the filling up to 100% of the mass was described.

The patent application P-381349 (publ. 11.06.2007) described into-intestinal dietetic composition containing alive bacteria cultures and products of plant origin **characterized in that** it contains probiotic bacteria especially *Lactobacillus plantarum* in quantity ≥ 1 x 10⁹ CFU/g in quantity from 10 to 30%, lecithin in quantity from 0 to 95% and defatted ground flaxseed in quantity from 5 to 95% in ratio to the total mass of the composition.

The patent application AT414095B (published 15.12.2005) described preparation (I) for the treatment of dry mucous membrane in the oral and throat area, which is accompanied by negative conditions e.g. swallowing difficulties, comprises at least a medically compatible and/or food organic substance; at least a medically compatible and/or food regulating certified flavor material; mucoadhesive substance; a sugar and sugar alcohol; and a carrier substance such as fat and/or oil and further a linking agent such as triglyceride and/or hydrophobic liquid, which exhibits lubricating action. Preparation for the treatment of dry mucous membrane in the oral and throat area, which is accompanied by negative conditions such as swallowing difficulties, foreign body sensation in the neck area and pharyngitis, comprises at least a medically compatible and/or food regulating organic substance (15-60 (preferably 20-25)%) such as citric acid, tartaric acid, maleic acid, gluconic acid, ascorbic acid, succinic acid or adipic acid, as a salivation promoting substance (5-25 (preferably 10-20)%); at least medically compatible and/or food regulating certified flavor material; at least a mucoadhesive substance (25-70 (preferably 50-65)%) such as modified cellulose, hydroxymethyl, hydroxyethyl, hydroxypropyl, hydroxypropylmethyl and sodiumcarboxymethylcellulose, polyvinylpyrrolidone, polyethyleneglycol, tragacanth, gum arabic, gelatin, agar-agar, polyacrylic acid and/or polycarbophil; a sugar and sugar alcohol such as glucose, fructose, mannose, maltose, sorbitol, xylitol, mannitol, and synthetic sweetener (saccharin, acesulfame, cyclamate, aspartame and/or its salts); and a carrier substance such as fat and/or oil (almond oil, peanut oil, olive oil, sesame oil, linseed oil, rapeseed oil, mawseed oil, behen oil, and/or neutral oil), and further a linking agent (triglyceride and/or hydrophobic liquid (silicone oil, liquid paraffins and/or low fatty acids (preferably isopropyl myristate, isopropyl palmitate, ethyl and/or oleyl oleate)), which exhibits lubricating action, where (I) is present as gel and in amount of 0.5-5 (preferably 2-3)%. Independent claims are included for: (1) a dental prosthesis-adhesive cream and/or hard gel comprising (I); and (2) a carrier substance of fat and/or oil useful in the manufacture of (I) for mouth area and disrupted area.

Despite the afore-mentioned solutions, and despite recognizing the causes of appearing and staying of bad breath, the problem of removing the fur from the tongue still exists, which is especially troublesome in difficult to access basal part of the tongue. This part is especially difficult to clean also with toothbrushes that have mechanical removing (frictional) elements.

The aim of the invention is providing a solution that would prevent forming the fur and appearing unpleasant odor, and which would mechanically, in gentle manner, clean the tongue, without use of chemical agents. Unexpectedly, by the use of particles of ground defatted and/or partially defatted and/or non-defatted flaxseed (*Linum usitatissimum L.)* hardened with caramel mass, such solution has been achieved in the invention.

The subject of the invention is the method of production of the product for sucking for cleaning the tongue **characterized in that** in caramel sugar or non-sugar mass defatted **and/or** partially defatted and/or non-defatted fragmented flaxseed is hardened, where the flaxseed form a pumice grater cleaning the tongue.

Preferably, when the flaxseed hardened in caramel mass comprise 0,1 - 20%.

Preferably when fragmented flaxseed is introduced to the process during cooking and/or directly after cooking caramel mass at the time of initial cooling but still in higher temperatures, and/or during cooling the mass, what leads to partial mucilaging of flaxseed.

Preferably, when flaxseed defatted and/or partially defatted of any granulation is added, preferably after sieving through a sieve of mesh 0,5 - 6 mm.

Preferably, when the product is based on sugar free mass, preferably based on isomalt and/or strong sweeteners, preferably aspartame and/or acesulfame K, or on the base typical for caramel mass containing sugar and/or glucose and/or honey and/or other known sweetening substances.

Preferably, when natural and/or synthetic flavorings and/or natural and/or synthetic colorings, preferably when mint and/or eucalyptus and/or lemon flavors are added.

Another subject of the invention is a food or pharmaceutical product for cleaning the tongue, **characterized in that** the rubbing agent in the form of fragmented flaxseed is hardened in sugar containing or sugar free caramel mass forming a delicate pumice grate that cleans the tongue from bacterial fur, stimulating saliva production and refreshing the breath, providing at the same time peeling and massage of the tongue and hindering the formation of bacterial fur on the tongue.

The food or pharmaceutical product for cleaning the tongue **characterized in that** the rubbing agent in the form of fragmented flaxseed is pressed with an addition of supplementary substances such as filling, bonding, lubricating, sliding, modifying the behavior in alimentary tract, colorants, and flavor improving substances.

Preferably, when the flaxseed are defatted and/or partially defatted and/or non-defatted ground flaxseed and that the flaxseed particles, hardened in caramel mass comprise 0.1 - 20% of the mass.

Preferably, when the product contains flaxseed defatted and/or partially defatted of any granulation is added, preferably after sieving through a sieve of mesh 0,5 - 6 mm.

Preferably, when it is produced on the base of typical caramel mass containing sugar and/or glucose and/or honey and/or other known sweetening substances, or it is produced on the sugar free bases, preferably based on isomalt and/or strong sweeteners, preferably aspartame and/or acesulfame K, and that it contains natural and/or synthetic flavorings and/or natural and/or synthetic colorings, preferably when mint and/or eucalyptus and/or lemon flavors.

Preferably, when the product contains flaxseed particles which enrich the product in the natural nutritional herbal component making a dietary supplement.

Preferably, when the product is produced in the form of lozenges, dragees, tablets, candies, gums, gummy candies or lollipops.

Another subject of the invention is using flaxseed hardened in sugar containing or sugar free caramel mass where defatted and/or partially defatted and/or non-defatted flaxseed form delicate pumice grate for cleaning the tongue.

Preferably, when flaxseed particles are hardened in caramel mass at 0.1 - 20% and/or flaxseed is partially mucilaged.

Preferably, when as the rubbing-cleaning agent where defatted and/or partially defatted and/or non-defatted flaxseed of any granulation is used, preferably after sieving through a sieve of mesh 0,5 - 6 mm, and/or hardened flaxseed particles which enrich the ready product in in the natural nutritional herbal component. Preferably, when the grate stimulates saliva production and refreshes the breath, providing at the same time peeling and massage of the tongue and hindering the formation of bacterial fur on the tongue and being a dietary supplement or a pharmaceutical product.

Examples of the invention application are presented below.

### EXAMPLES

### Example 1

Sugar free caramel mass according to the usual/known method based on 94% isomalt with the addition of strong sweeteners: 0,04% acesulfame K and 0,04% aspartame E and water was prepared. Directly after cooking, to the hot caramel mass, 3,96% of ground flaxseed was added. The flaxseed, before adding, had been partially defatted to the fat content at 8% and sieved with a sieve of 1 mm mesh for uniform granulation. Also coloring substance was added: pistachio green (E104 + E 132). The mass was then seasoned with refreshing flavorings: per each 100 g of the product - eucalyptus oil 0,14 g, menthol 0,11 g and mint oil 0,08 g. After cooling the mass was formed into candies for sucking of 3,5 g. The candies were packed in blisters according to the usual/known methods.

### Example 2

Caramel mass was prepared from white sugar 40%, glucose syrup 37% with addition of condensed milk 14% and solidified plant fat 2% and water. To the hot caramel mass directly after cooking, ground defatted flaxseed sieved through a sieve of 2 mm mesh in the amount of 0,8% was added, then the mass was cooled while seasoning and coloring done at the same time (mint oil, menthol, eucalyptus oil in ratio 1: 1,5: 1,8) - 1,32 g/100 g, salt 0,034% and coloring pistachio green 0,007%) then formed according to known method in lollipops and mounted on sticks.

### Example 3

Caramel mass made from white sugar 55%, glucose syrup 35% with addition of honey 2% and water was prepared. To the hot caramel mass, directly after cooking, ground flaxseed sieved through a sieve of 1 mm mesh in the amount of 6% was added, and coloring pistachio green (E 104 + E 132) 0,007%, then the mass was cooled while seasoning done at the same time (with mixture of mint oil, menthol, eucalyptus oil in ratio 1: 1,5: 1,8 - 1,32 g/100 g) and formed in dragees/drops of 2,5 - 3,5 g.

### Example 4

Sugar free caramel mass according to the usual/known method based on 94% isomalt with the addition of strong sweeteners: 0,04% acesulfame K and 0,04% aspartame E and 1,5% partially defatted coarsely ground flaxseed and water. The remains of fat in the partially defatted flaxseed were 8%. Directly after cooking to the caramel mass colorings were added, then the mass was cooled according to the usual/known method and seasoned with refreshing flavors: mint oil, menthol, eucalyptus oil, then was formed in dragees/drops of 2,5 g and packed in blisters coated with aluminum foil.

### Example 5

Sugar free caramel mass according to the usual/known method based on 94% isomalt with the addition of strong sweeteners: 0,04% acesulfame K and 0,04% aspartame E and water. Directly after cooking, to the caramel mass, colorings were added, then the mass was preliminarily cooled. Then ground flaxseed in the amount of 5% was added and then also refreshing flavors: mint oil, menthol, eucalyptus oil with concurrent further cooling of the mass according to the usual/known method and then forming flat drops of 2,5 g.

### Example 6

Caramel mass made from white sugar 54%, glucose syrup 34% and water was prepared. To the hot caramel mass, directly after cooking, ground defatted flaxseed (the oil content to 11%) sieved through a sieve of 3 mm mesh in the amount of 8% was added, then coloring pistachio green (E 104 + E 132) 0,015%, then the mass was cooled while seasoned at the time with flavor mixture of mint oil, menthol, eucalyptus oil in ratio 1: 1,5: 1,8 in the amount of - 1,32 g/100 g and formed in drops/candies of any shape.

### Example 7

Water sugar-syrup solution was prepared according to the known/usual method with addition of gelatin, solidified plant fat, emulgators and colorings with addition of partially defatted (the oil content to 9%) and ground. directly after cooking citric acid and flavors were added. The mass was aired, cooled and formed into a cuboid with concurrent folding of each candy/chewing gum.

### Example 8

Caramel mass was prepared according to the known/usual method from granulated saccharose and liquid glucose (50:50), propylene glycol, caramel and water. At the end of preparing the mass, as an active substance, 10% of ground flaxseed was added (*lac. Linum usitatissimum L. - Lini semen*) defatted earlier to the oil content of 11 % and sieved with a sieve of 3 mm mesh for uniform granulation, and beta carotene as coloring and supplementing substance was added in the amount of 5%. In the initial stage of cooling the mass partial release and hardening of flaxseed mucilage occurred. Then the mass was cooled while seasoned with citric acid and formed into lozenges to be sucked to obtain a local effect.

The addition of nutritional flaxseed as an unconventional component of pumice-like drops/candies for cleaning the tongue further emphasizes the value of the ready product based on the invention.

Candies for cleaning the tongue contain particles of flaxseed, which is one of the richest known sources of soluble and non-soluble dietary fibre, exogenic aminoacids, and antioxidants such as lignans, phenolic acid, and flavonoids, also rich in macro- and micro-elements. Some nutritional components in flaxseed, e.g. antioxidant lignans are considered (according to pre-clinical and clinical trials) as a group of dietary components of potential anti-cancer and phyto-hormone activity [Bommareddy A., Arasada BL, Mathees DP, Dwidedi C. Chemoprotective effect of dietary flaxseed on colon tumor development. Nutr cancer: 2006: 54(2):216-22*;* Nesbitt PD, Lam Y., Thompson LU. Human metabolizm of lignan precursors in raw and processed flaxseed. Am J Clin Nutr. 1999; 69(3):549-5]*.*

## Claims

1. A method of production of the product for cleaning the tongue, **characterized in that** in sugar containing or sugar-free caramel mass a rubbing agent is hardened in the form of flaxseed and pumice-like grate cleaning the tongue is formed.

2. A method according to claim 1, **characterized in that** the flaxseed is defatted and/or partially defatted and/or non-defatted, and that the flaxseed particles hardened in caramel mass comprise 0,1 - 20% of the mass.

3. A method according to claim 1 or 2, **characterized in that** the ground flaxseed is introduced to the process during cooking and/or directly after cooking the caramel mass at the stage of preliminary cooling, but still in higher temperatures, and/or during cooling the mass, leading to partial mucilaging of the mass.

4. A method according to claim 1 or 2, **characterized in that** flaxseed defatted and/or partially defatted of any granulation is added, preferably after sieving through a sieve of 0,5 - 6 mm mesh.

5. A method according to claim 1 or 2, **characterized in that** the product is produced on the sugar-free bases, preferably on the base of isomalt and/or strong sweeteners, preferably aspartame and/or acesulfame K, or on the base of typical caramel mass containing sugar and/or glucose and/or honey and other known sweetening substances, and preferably natural and/or synthetic flavor and/or natural and/or synthetic colorings are added, preferably mint and/or eucalyptus and/or lemon flavors.

6. A product for cleaning the tongue, **characterized in that** the rubbing agent in the form of ground flaxseed is hardened in sugar containing or sugar free caramel mass, forming a delicate pumice-like grate cleaning the tongue of bacterial fur, stimulating saliva production, refreshing breath, providing at the same time peeling and massage of the tongue and hindering formation of bacterial fur on the tongue.

7. A product according to claim 6, **characterized in that** the flaxseed is defatted and/or partially defatted and/or non-defatted ground flaxseed, and that the flaxseed particles are hardened in caramel mass comprise 0,1 - 20% of the mass.

8. A product according to claim 6 or 7, **characterized in that** it contains ground flaxseed defatted and/or partially defatted of any granulation is added, preferably after sieving through a sieve of 0,5 - 6 mm mesh.

9. A product according to claim 6 or 7, **characterized in that** it is produced on the base of typical caramel mass containing sugar and/or glucose and/or honey and other known sweetening substances, or is produced on the sugar-free bases, preferably on the base of isomalt and/or strong sweeteners, preferably aspartame and/or acesulfame K, and it contains natural and/or synthetic flavors and/or natural and/or synthetic colorings are added, preferably mint and/or eucalyptus and/or lemon flavors.

10. A product according to claim 6 or 7, **characterized in that** it contains flaxseed particles, which enrich the product in natural nutritional herbal component with physiological effect and is a dietary supplement or a pharmaceutical product.

11. The product according to claim 6 or 7, **characterized in that** it is in the form of pills for sucking and chewing, drops, dragees, candies, gums, gummy candies, lollipops, lozenges or tablets.

12. Use of flaxseed hardened in sugar containing or sugar free caramel mass in which defatted and/or partially defatted and/or non-defatted ground flaxseed form a delicate pumice-like grate, for producing the product for cleaning the tongue.

13. Use according to claim 12, wherein flaxseed particles are hardened in caramel mass in the amount of 0,1 - 20% and/or flaxseed is partially mucilaged.

14. Use according to claim 12, wherein as the rubbing component, ground flaxseed, defatted and/or partially defatted and/or non-defatted of any granulation is added, preferably after sieving through a sieve of 0,1 - 6 mm mesh and/or hardened flaxseed particles enrich the ready product in natural nutritional herbal component.

15. Use according to claim 12, wherein the grate cleaning the tongue from bacterial fur, also stimulates saliva production, refreshes breath, providing at the same time peeling and massage of the tongue and hinders formation of bacterial fur on the tongue and is a dietary supplement or a pharmaceutical product.
